# EUROPEAN PATENT SPECIFICATION

(11) **EP 0 279 319 B1**
(45) Date of publication and mention of the grant of the patent: **13.07.1994**
(21) Application number: 88101817.0
(22) Date of filing: 08.02.1988
(51) Int. Cl.: A61K 7/021

(54) **Cosmetic compositions comprising oil-in-water emulsion containing pigment**
Kosmetische Präparate mit pigmenthaltiger Öl-in-Wasser-Emulsion
Compositions cosmétiques comprenant une émulsion huile-dans-l'eau contenant du pigment

(30) Priority: 06.02.1987 US 11971
(43) Date of publication of application: 24.08.1988
(73) Proprietor: Revlon Consumer Products Corporation, New York, NY 10022 (US)
(72) Inventor: Brieva, Hernando, Manalapan New Jersey (US); Jose, Natividad, Jamaica New York (US); Tietjen, Marlene, New York New York (US)
(74) Representative: Sanderson, Laurence Andrew

(56) References cited:
- EP-A- 0 133 963
- EP-A- 0 154 837
- EP-A- 0 212 870
- PATENT ABSTRACTS OF JAPAN, vol. 60, no. 11 (C-405), 24th February 1987; & JP-A-61 218 509 (SHISEIDO) 29-09-1986

## Description

The present invention relates to cosmetic compositions in general and more specifically to compositions of an oil-in-water emulsion containing other cosmetically desirable components and pigment.

The present invention comprises a cosmetic composition which is an oil-in-water emulsion comprising
(a) an oil phase which comprises
   (i) a coated pigment consisting essentially of finely divided particles of pigment (inorganic or organic) whose surfaces are chemically bonded to, and physically completely coated by, polysiloxane which coating renders the particles hydrophobic, and
   (ii) a silicone component selected from the group consisting of dimethyl polysiloxane having the formula : (CH₃)₃SiO(Si(CH₃)₂O)_{d}-Si(CH₃)₃ wherein the degree of polymerization d is effective to give the fluid a viscosity of 0.65 to one million mm²/S (centistokes) at 25° C; cyclomethicone having a degree of polymerization of 3 to 6; organopolysiloxane having the formula

      X(CH₃)₂SiO-Y-Si(CH₃)₂X

      wherein X is alkyl or alkoxy having 1 to 30 carbon atoms and Y is a chain of 1 to 100 repeating (Si-O) units containing 1 to 100 units of the formula (-Si(R₁)(R₂)O-) and 0 to 100 units of the formula (Si(R₃)(R₄)O) wherein each of R₁, R₂, R₃ and R₄ can be alkyl containing 2 to 30 carbon atoms, phenyl, or phenyl connected to the Si atom by a vinyl group or an alkylene bridge 1 to 3 carbon atoms long; wherein each R₁ and R₃ can also be -CH₃, and each R₁ and R₂ can also be trimethylsiloxy; and mixtures thereof;
(b) an aqueous phase;
(c) a surfactant which comprises one or more
   polydiorganosiloxane-polyoxyalkylene copolymers containing at least one polydiorganosiloxane segment consisting of

   R_{b}SiO_{(4-b)/2}

   siloxane units wherein b has a value of from 0 to 3 inclusive, there being an average of approximately two R radicals per silicon in the copolymer, and wherein R denotes a radical selected from the group consisting of methyl, ethyl, vinyl, phenyl and a divalent radical of 2-6 carbons bonding a polyoxyalkylene segment to the polydiorganosiloxane segment, at least 95 percent of all R radicals being methyl; and containing at least one polyoxyalkylene segment having an average molecular weight of less than 5000 and consisting of from 0 to 50 mol percent polyoxypropylene units and from 50 to 100 mol percent polyoxyethylene units, at least one terminal portion of said polyoxyalkylene segment being bonded to said polydiorganosiloxane segment, any terminal portion of said polyoxyalkylene segment not bonded to said polydiorganosiloxane segment being satisfied by a terminating radical;
(d) optionally a second organic surfactant which is silicone-free;
   wherein the surfactant component or components are present in an amount effective to form a stable emulsion of said oil phase in said water phase.

The cosmetic compositions of the present invention are useful in a variety of makeup products such as foundations, eyeshadows, blushers, and the like. The invention permits incorporation of pigment into the oil phase of oil-in-water emulsions, resulting in fluid products with excellent blending capability. In particular, the present invention avoids the problem known as "setting", in which the attempt to blend high amounts of pigment into the water phase leads to a product which cannot be applied in a satisfactory manner and results in an uneven, draggy, streaky appearance upon application to the skin. Thus, the present invention permits incorporation of higher amounts of pigment than previously thought possible in oil-in-water emulsion products.

The oil phase comprises 10 to 80 weight percent of the composition, preferably 10 to 50 weight percent thereof. It contains as the major component one or both of dimethyl polysiloxane having the chemical formula (1)

(CH₃)₃SiO(Si(CH₃)₂O)_{d}-Si(CH₃)₃ (1)

in which the degree of polymerization d has a value, typically between 1 and 4160, effective to give the fluid a viscosity of 0.65 to one million mm²/S (centistokes) at 25° C; and/or a cyclomethicone having D= 3 to 6 repeating units of formula (2):

-(Si(CH₃)₂O)_{D}- (2)

The combined amount(s) of dimethyl polysiloxane, cyclomethicone, and organonolysiloxane of the formula X(CH₃)₂SiO-Y-Si(CH₃)₂X preferably comprise about 10 to about 90 weight percent of the emulsion.

The hydrophobic coated pigments useful in the present invention have one of the following formulae (3) to (5):

P-[-0-Si-[-(0SiA₂)₀₋₁₀₀-A]₃]₁₋₁₀₀ (3)

wherein each of the oxygen atoms at the left end of formula (3) is attached to an atom P in the pigment surface; and each A is an alkyl or alkenyl group having up to 30 carbon atoms. A number of adjacent polysiloxane chains as shown in formula (3) can be cross-linked through oxygen atoms to form a polysiloxane chain with up to 100 repeating -Si(-OP)-O- units that extends along the pigment surface, in addition to the polysiloxane chain which extends away from the pigment surface. Examples of alkyl groups are methyl, ethyl, propyl, butyl, pentyl, hexyl, heptyl, octyl, and so forth up to octadecyl. "Alkenyl" means carbon chains with one or more double bonds; examples of such groups include ethylene, propylene, acrylyl, methacrylyl, and residues of unsaturated fatty acids such as oleic (C₁₇H₃₃-), linoleic (C₁₇H₃₁-), and linolenic (C₁₇H₂₉-).

The coated pigments can also exhibit structural formula (4):

P-O-(Si(CH₃)₂O)ₚ-Si(CH₃)₃ (4)

wherein p is 1-100, and P is an atom in the pigment surface.

The coated pigments can also exhibit structural formula (5):

(CH₃)₃SiO-[Si(CH₃)(-OP)-O-Si(CH₃)(-O-P)-O-]₁₋₅₀-Si(CH₃)₃ (5)

wherein P is an atom in the pigment surface, and in which each of the up to 100 repeating (Si-O) units is bonded through an oxygen atom to the pigment surface.

The number of polysiloxane chains of formulas (3), (4), and (5) that are bonded to the pigment surface is not known but is sufficiently high to coat the pigment completely and render it completely hydrophobic. Hydrophobicity can readity be determined by placing the coated pigment into water and observing whether any becomes dispersed of suspended in the water.

Suitable pigments include all inorganic and organic pigments of fillers which are usable in cosmetic formulations. Particular examples include talc, mica, titanium dioxide, ferric oxide, ferrous oxide, kaolin, ultramarine, chromium oxide, chromium hydroxide, zinc oxide, silica, manganese violet, and their equivalents. Other examples include lakes of organic colorants such as FD & C Red No. 7 calcium lake, FD & C Yellow No. 5 aluminum lake, D & C Red No. 9 barium lake, and D & C Red No. 30.

The pigment (or a mixture of two or more pigments) can be coated by placing it in dry, finely divided form in a mixer and adding a silicone material selected from (A) and/or (B) and/or (C) as defined below:
(A) is A₁SiX₁X₂X₃, wherein A is an alkyl or alkenyl group having 1 to 30 carbon atoms, and X₁, X₂ and X₃ are independently chloro, methoxy, or ethoxy (this material will form coated pigment having formula (3));
(B) is material of the formula

   (CH₃)₃SiO-(Si(CH₃)₂O)ₚ-Si(CH₃)₂OA₂

   wherein p is 1 to 100, and A₂ is hydrogen or an alkyl group having 1 to 30 carbon atoms (this material will form coated pigment having formula (4));
(C) is material of the formula

   (CH₃)₃SiO(Si(CH₃)(H)-O)ᵢ-Si(CH₃)₃

   wherein i is 1 to 100 (this material will form coated pigment having formula (5)); or a one-phase mixture of two or all three of A, B, and C. The relative amounts of fluid: pigment should be sufficient to coat the pigment particles. Generally a fluid: pigment weight ratio is satisfactory for which 1-4 weight percent of the final product is silicone. The pigment and fluid are intimately mixed thoroughly to obtain a uniform dispersion of the fluid on the pigment, in which the fluid completely coats the particles of pigment. The slurrying operation is advantageously carried out at a temperature of 25° C to 160°C effective to promote hydrolysis and reaction of the silicone with the pigment. As an alternative to synthesis, satisfactory coated pigments usable in this invention are commercially available from a variety of sources.

The coated pigment comprises 2 to 40 weight percent, and preferably 5 to 30 weight percent, of the emulsion.

The oil phase may also contain optional cosmetically acceptable oil-soluble components, such as preservatives, (e.g. up to 0.5 weight percent of the oil phase of paraben such as propyl parabenl; up to 1 percent by weight of the oil phase of any conventional cosmetically acceptable fragrance; and one or more of other components well-known to cosmetic chemists, such as those that are intended for cosmetic purposes, for skin-softening and/or for physiological purposes, e.g. for treating skin conditions, like dry skin or chapped skin.

Examples of oil-soluble personal-care components that are useful in the compositions of this invention include, but are not limited to ester waxes, oils and fats of animal or vegetable origin, such as spermaceti wax; beeswax, carnauba wax, lanolin wax, coconut oil, castor oil and lanolin oil; fatty alcohols such as cetyl alcohol, stearyl alcohol and lauryl alcohol; fatty acids such as stearic acid and palmitic acid; alkyl eaters of fatty acids such as the methyl, ethyl or isopropyl ester of said fatty acid; hydrocarbon oils and waxes such as mineral oil, petrolatum, perhydrosqualene and paraffin wax; and sunscreens such as octyldimethyl-PABA (PABA=4-amino-benzoic acid).

By "surfactant" is meant one or more than one surfactant compound as defined hereinbelow, provided that the compound or mixture of compounds used has the desired emulsion-stabilizing properties described herein. The surfactant must be capable of forming a stable oil-in-water emulsion, which means it will exhibit an HLB (hydrophile-lipophile balance) value of about 6 to about 18, preferably about 10 to about 14.

The surfactant includes one or more polydiorganosiloxaneoxyalkylene compounds. The polydiorganosiloxane segments of this surfactant consist of siloxane units which are interlinked by Si-0-Si linkages and which have the formula

R_{b}SiO_{(4-b)/2}

The value of b may range from 0 to 3 for said siloxane units with the proviso that there is an average of approximately 2, i.e. from 1.9 to 2.1 R radicals for every silicon atom in the copolymer. Suitable siloxane units thus include R₃SiO_{1/2}, R₂SiO_{2/2}, RSiO_{3/2}, and SiO_{4/2} siloxane units taken in such molar amounts so that b has an average value of approximately 2 in the copolymer. Said siloxane units may be arranged in linear, cyclic and/or branched fashion.

The R radicals of this surfactant may be any radical selected from the group consisting of methyl, ethyl, vinyl, phenyl, and a divalent radical bonding a polyoxyalkylene segment to the polydiorganosiloxane segment. At least 95 percent of all R radicals are methyl radicals; preferably there is at least one methyl radical bonded to each silicon atom. Divalent R radicals preferably contain no more than 6 carbon atoms. Examples of divalent R radicals include -O-, -CₘH₂ₘO-, -CₘH₂ₘ- and -CₘH₂ₘCO₂- where m is an integer greater than zero.

Illustrative of the siloxane units that make up the polydiorganosiloxane segments of this first surfactant are the following, where Me denotes methyl and Q denotes said divalent R radical together with its bonded polyoxyalkylene segment:
R₃SiO_{1/2} units such as Me₃SiO_{1/2},
Me₂(CH₂=CH)SiO_{1/2}, Me₂(C₆H₅)SiO_{1/2},
Me(C₆H₅)(CH₂=CH)SiO_{1/2}, Me₂(CH₃CH₂)SiO_{1/2},
Me₂QSiO_{1/2}, MeQ₂SiO_{1/2}, Q₃SiO_{1/2},
Q₂(CH₃CH₂)SiO_{1/2}, and Me(C₆H₅)(Q)SiO_{1/2};
R₂SiO_{2/2} units such as Me₂SiO_{2/2}, Me(C₆H₅)SiO_{2/2},
Me(CH₂=CH)SiO_{2/2}, (C₆H₅)₂SiO_{2/2}, MeQSiO_{2/2}, and
Q(C₆H₅)SiO_{2/2}; RSiO_{3/2} units such as MeSiO_{3/2},
C₆H₅SiO_{3/2}, CH₂=CHSiO_{3/2}, CH₃CH₂SiO_{3/2} and
QSiO_{3/2}; and SiO_{4/2} units.

It is to be understood that the silicone-based surfactant may comprise one or more of said polydiorganosiloxane segments. The number of and average molecular weight of the polydiorganosiloxane segments in the copolymer is related to the desired weight ratio, hereinafter described, of the polysiloxane and polyoxyalkylene segments in the polymer. Preferably it comprises one polydiorganosiloxane segment having bonded thereto one or more polyoxyalkylene segments.

The polyoxyalkylene segments of this surfactant consist of oxyethylene units of the formula -CH₂CH₂O-, alone, or in combination with oxypropylene units of the formula -CH₂CH(CH₃)O-, an average of at least half of the oxyalkylene units in the polyoxyalkylene segments being oxyethylene units. Suitable emulsions of this invention are not formed when the polyoxyalkylene segments contain more than 50 mol percent of the relatively hydrophobic oxypropylene unit. The polyoxyalkylene segments thus correspond to the formula (-CH₂CH₂O-)ₚ(-CH₂CH(CH₃)O-)_{q} wherein the oxyalkylene units may be arranged in any suitable fashion such as random, alternating and block. The average values of p and q are such that the value of p is equal to, or greater than, the value of q and the sum of p+q is sufficient to provide an average molecular weight of under 5,000. Preferably, the value of p exceeds q.

The polyoxyalkylene segments are bonded to the polydiorganosiloxane segments by at least one terminal portion of said polyoxyalkylene segment, said bonding being by way of a divalent R radical, hereinbefore described. It is to be understood that said bonding may be by both terminal portions of said polyoxyalkylene segment in those copolymers comprising more than one polydiorganosiloxane segments. Any terminal portion of the polyoxyalkylene segment that is not bonded to a polydiorganosiloxane segment is satisfied by a terminating radical. The type of said terminating radical is not critical and may be monovalent, thereby terminating one polyoxyalkylene segment, or polyvalent, thereby terminating more than one polyoxyalkylene segment. Said terminating radicals are made up of atoms selected from the group consisting of carbon, hydrogen, nitrogen and oxygen. Illustrative of said terminating radicals are hydrogen; hydroxyl, alkyl, such as methyl, ethyl, propyl, butyl, benzyl, aryl such as phenyl; alkoxy such as methoxy, ethoxy, propoxy, butoxy, benzyloxy; aryloxy, such as phenoxy; alkenyloxy, such as vinyloxy and allyloxy; acyloxy, such as phenoxy; alkenyloxy, such as vinyloxy and allyloxy; acyloxy, such as acetoxy, acryloxy and propionoxy; and amino such as dimethylamino.

Herein "copolymer" means either a block arrangement of segments such as denoted by the formulae (AB)_{c}, A(BA)_{c} and B(AB)_{c} or a pendant arrangement of segments such as (AB_{d})_{c} or combinations thereof wherein A denotes a polydiorganosiloxane segment, B denotes a polyoxyalkylene segment and c and d denote integers greater than zero and greater than one, respectively. Copolymers may be prepared by modification of the well-known methods described in the polydiorganosiloxane-polyoxyalkylene copolymer art. The following patents are hereby incorporated herein by reference to show the preparation of polydiorganosiloxane-polyoxyalkylene copolymers: Haluska, U.S. Pat. No. 2,868,824; Haluska, U.S. Pat. No. Re. 25,727; Bailey, U.S. Pat. No. 3,172,899; Pater, U.S. Pat. No. 1,234,252; Simmler, et al. U.S. Pat. No. 3,174,987: Bailev, et al., U.S. Pat. Nos. 3,562,786, 3,600,418 and 3,629,308; Holdstock, U.S. Pat. No. 3,629,165; and Gee et al., U.S. Pat. No. 4,122,029.

It is to be understood that the silicon-bonded reaction groups such as silicon-bonded hydrogen for addition reactions or silicon-bonded hydrolyzable radicals for displacement reactions are preferably completely reacted in the copolymer preparation process, but that trace amounts of said reaction groups may escape reaction with the polyoxyalkylene and may be found in the surfactant.

Non-essential components which are common to personal-care compositions of the art, such an perfumes, humectants, preservatives, colourants and electrolytes may be incorporated into the compositions of this invention provided they do not destablize the emulsion so as to cause a breaking or an inverting of the emulsion.

The cosmetic composition of the present invention can optionally further comprise a second surfactant which is organic, silicone-free, and has on HLB value provided that the overall effective HLB value of all surfactants present (preferably about 10 to about 14) still permits formation of the desired oil-in-water emulsion. The total amount of surfactant will typically be about 0.5 to 10 weight percent of the composition. As is well known to those of ordinary skill in this art, the HLB value is determined by a standardized technique for measuring the solubility of a surfactant. The second surfactant may be anionic, cationic or non-ionic with respect to its hydrophilic portion.

Satisfactory second surfactants useful in this invention include "Tween 20" (HLB 16.7); "Arlacel 20" (HLB 8.7); and "Solulan 98" (HLB 13). Other examples of suitable surfactants include sodium capryl lactylate and sodium stearoyl lactylate as anionic surfactants, quaternary ammonium chlorides manufactured by Tomah Products, Inc. as Emulsifier Three (TM) and Emulsifier Four (TM) as cationic surfactants and polyethylene glycol (200) monolaurate, glycerol monolaurate, N,N-dimethylcaproamide, diethylene glycol monolaurate, sorbitan monolaurate and nonylphenoxy polyethoxyethanol as non-ionic surfactants. Other satisfactory silicone-free surfactants include the following: glyceryl monooleate, polyglyceryl-4 decaoleate, PEG-8 Oleate, PEG-4 lauryl ether, and PEG-9 lauryl ether. Other examples of suitable second surfactants having the proper HLB value may be found by reference to standard publications such am McCutcheon's, Detergents and Emulsifiers, Allred Publishing Company, Ridgewood, N.J. (1974).

The water phase of the composition of the present invention preferably comprises 25 to 80 weight percent of the emulsion. This phase may be simply water, or may contain water-soluble cosmetically acceptable components provided that the emulsion is not destabilized or inverted therein. Examples include ethanol; isopropanol; humectants, including glycerol, propylene glycol, sodium pyrrolidone carboxylic acids and citric acid, lactic acid and derivatives thereof; preservatives, such as methyl paraben; electrolytes, such as NaCl and magnesium sulfate; and sunscreens such as TEA-salicylate or PABA. The water phase comprises up to about 80 weight percent, preferably up to about 60 weight percent and more preferably up to about 50 weight percent, of the composition, and at least enought water to permit emulsification of the non-aqueous components, preferably at least about 25 weight percent of the composition.

The composition can also contain effective amounts of optional cosmetically acceptable thickeners or other components such as cellulose derivatives, clays and organically modified clays, and organic thickeners to achieve desired properties such as viscosity, stability or afterfeel. Specific examples are the following: sodium carboxymethyl cellulose, magnesium aluminum silicate, bentonite, hydroxyethyl cellulose, xanthan gum, cationic cellulosic resins, quaternium-18 hectorite, and glyceryl trihydroxy stearate. These modifiers can be added to the aqueous or non-aqueous phase of the composition.

To make the composition of the present invention, one (1) stirs thoroughly together all the components of the oil phase (the surfactant(s) are added to the phase (oil or water) in which they are more soluble), (2) disperses the hydrophobic pigments in the oil phase utilizing a high speed disperser or high shear mill and then (3) stirs the oil phase into the water phase including any components dissolved in the water phase. Any standard high speed stirring or homogenizing apparatus known to the art can be used to carry out the mixing operation. Preferably, components that are solid at room temperature (e.g. waxes), are heated to soften or liquify them prior to mixing with the other liquid components.

The invention will be described further in the following examples, which should be interpreted as illustrative rather than limiting. In each example, the indicated ingredients were combined into an "oil phase" mixture and a "water phase" mixture. The two mixtures were then thoroughly mixed together as described above, to produce a cosmetic product which was an oil-in-water emulsion having superior applicability, and non-dragging, non-streaking and non-setting properties.

### EXAMPLE 1

| Component | Amount (% by weight) |
|---|---|
| Oil Phase | |
| Dimethicone (10 mm²/S (centistokes)) | 15.0 |
| Cyclomethicone (D=5) | 5.0 |
| Surfactant (Union Carbide "Silwet L7500" dimethicone copolyol, HLB=10) | 1.0 |
| Pigment* | 15.0 |

| Aqueous Phase | |
|---|---|
| Water | 63.0 |
| Surfactant (Dow Corning 193 dimethicone copolyol, HLB=13.6) | 1.0 |

| | |
|---|---|
| *:The pigment had been thoroughly coated with a polymethyl hydrogen siloxane coating bonded to the pigment surface. | |

### EXAMPLE 2

| Components | Amount (% by weight) |
|---|---|
| Oil Phase | |
| Phenyl dimethicone (Dow Corning 556 fluid) | 15.0 |
| Dimethicone (viscosity 60 mm²/S (cs)) | 8.0 |
| Surfactant (Silwet L7500, HLB = 10) | 1.5 |
| Pigment* | 20.0 |

| Water Phase | |
|---|---|
| Surfactant (Dow Corning 193, HLB = 13.6) | 1.2 |
| Xanthan gum | 0.5 |
| Water | 53.8 |

| | |
|---|---|
| *Pigment was coated with polymethyl hydrogen siloxane bonded to the pigment surface. | |

### EXAMPLE 3

| Component | Amount (% by weight) |
|---|---|
| Oil Phase | |
| Phenyldimethicone | 15.0 |
| Cyclomethicone (D=5) | 15.0 |
| Mineral oil (viscosity= 70 mm²/S (cs)) | 5.0 |
| Surfactant (Silwet L7500, HLB=10) | 1.0 |

| Pigment* | |
|---|---|
| Red and black iron oxides | 3.5 |
| Talc | 2.5 |
| Ti0₂ | 10.0 |
| Sorbitan laurate (HLB=8.7) | 0.5 |

| Water Phase | |
|---|---|
| Water | 40.0 |
| Magnesium aluminum silicate | 1.0 |
| Surfactant (Union Carbide Silwet L7001, HLB=16) | 1.5 |
| Propylene Glycol | 5.0 |

| | |
|---|---|
| *The pigment was coated with methyl trimethoxy silane to form a coating of polydimethyl siloxane bonded to the pigment surface. | |

### EXAMPLE 4

| Component | Amount (% by weight) |
|---|---|
| Oil Phase | |
| Dimethicone (10 mm²/S (cs)) | 10.0 |
| Cyclomethicone (D=4) | 5.0 |
| Mineral oil | 10.0 |

| Pigment* | |
|---|---|
| Black, Yellow and Red Iron Oxides | 4.0 |
| TiO₂ | 16.0 |
| Talc | 5.0 |

| Water phase | |
|---|---|
| Surfactant (Dow Corning 193 fluid, HLB=13.6) | 5.0 |
| Water | 39.5 |
| Ethanol | 5.0 |
| Xanthan gum | 0.5 |

| | |
|---|---|
| *Pigment was coated with polymethylhydrogen siloxane bonded to the pigment surface and mineral oil. | |

### EXAMPLE 5

| Component | Amount (% by weight) |
|---|---|
| Oil Phase | |
| Pigment* | |
| Iron Oxide | 8.00 |
| TiO₂ | 7.00 |
| Dimethicone (10 mm²/S (centistokes)) | 12.00 |
| Cetyl alcohol | 2.0 |
| Stearyl dimethicone | 4.0 |
| Glycerol monolaurate | 0.50 |
| Bentonite | 2.0 |
| Preservative | 0.50 |

| Water Phase | |
|---|---|
| Water | 59.6 |
| Surfactant (Dimethicone copolyol, HLB = 13.6) | 5.0 |

| | |
|---|---|
| *:Pigment was coated as in Example 1. | |

### EXAMPLE 6

| Component | Amount (% by weight) |
|---|---|
| Oil Phase | |
| Pigment* | |
| FD&C Red #7 | 5.00 |
| FD&C Yellow #5 | 5.00 |
| Iron Oxide | 10.00 |
| Talc | 10.00 |
| Surfactant (Silwet L7500, HLB=10) | 3.00 |
| Surfactant (Silwet L722, HLB=9) | 1.00 |
| Dimethicone (100 mm²/S (centistokes)) | 10.00 |
| Cyclomethicone D=5 | 15.00 |
| Glyerol tribehenate | 8.00 |
| Preservative | 0.50 |

| Water Phase | |
|---|---|
| Water | 30.50 |
| Surfactant (Dow Corning 193 dimethicone copolyol, HLB=13.6) | 2.00 |

| | |
|---|---|
| *:Pigment was coated as in Example 1. | |

### EXAMPLE 7

| Component | Amount (% by weight) |
|---|---|
| Oil Phase | |
| Surfactant (Silwet L7500, HLB=10) | 2.50 |

| Pigment* | |
|---|---|
| Chromium Hydroxide Green | 10.00 |
| Ultramarine | 8.00 |
| titanated micas | 10.00 |
| Ceresin | 5.00 |
| Dimethicone (100 mm²/S (centistokes)) | 10.00 |
| Cyclomethicone 80% D=5, 20% D=4 | 15.00 |
| Polymethyloctadecyl siloxane | 5.00 |
| Preservative | 0.50 |

| Water Phase | |
|---|---|
| Water | 31.50 |
| Surfactant (Dow Corning, 193 HLB=13.6) | 2.5 |

| | |
|---|---|
| *:Pigment was coated as in Example 1. | |

## Claims

1. A cosmetic composition which is an oil-in-water emulsion comprising
a) an oil phase which comprises
i) a coated pigment consisting essentially of finely divided particles of pigment whose surfaces are chemically bonded to, and physically completely coated by, polysiloxane which coating renders the particles hydrophobic, and
ii) a silicone component selected from a dimethyl polysiloxane having the formula (CH₃)₃SiO(Si(CH₃)₂O)_{d}-Si(CH₃)₃ wherein the degree of polymerization d is effective to give the fluid a viscosity of 0.65 to one million mm²/S (centistokes) at 25°C; and/or cyclomethicone having a degree (D) of polymerization of 3 to 6 repeating units of the formula -(Si(CH₃)₂O)_{D}-; and/or organopolysiloxane having the formula
X(CH₃)₂SiO-Y-Si(CH₃)₂X
wherein X is alkyl or alkoxy having 1 to 30 carbon atoms and Y is a chain of 1 to 100 repeating (Si-O) units containing 1 to 100 units of the formula (-Si(R₁)(R₂)O-) and 0 to 100 units of the formula (Si(R₃)(R₄)O) wherein each of R₁, R₂, R₃ and R₄ can be alkyl containing 2 to 30 carbon atoms, phenyl, or phenyl connected to the Si atom by a vinyl group of an alkylene bridge 1 to 3 carbon atoms long; wherein each R₁ and R₃ can also be -CH₃, and each R₁ and R₂ can also be trimethylsiloxy; and mixtures thereof;
b) an aqueous phase;
c) a surfactant component which comprises one or more polydiorganosiloxane-polyoxyalkylene copolymers containing at least one polydiorganosiloxane segment consisting of siloxane units having the formula:
R_{b} SiO_{(4-b)/2}
wherein b has a value of from 0 to 3 inclusive, there being an average of approximately two R radicals per silicon in the copolymer, and R denotes a radical selected from methyl, ethyl, vinyl, phenyl or a divalent radical of 2-6 carbons bonding a polyoxyalkylene segment to the polydiorganosiloxane segment, at least 95 percent of all R radicals being methyl; and containing at least one polyoxyalkylene segment having an average molecular weight of less than 5000 and consisting of from 0 to 50 mol percent polyoxypropylene units and from 50 to 100 mol percent polyoxyethylene units, at least one terminal portion of said polyoxyalkylene segment being bonded to said polydiorganosiloxane segment, any terminal portion of said polyoxyalkylene segment not bonded to said polydiorganosiloxane segment being satisfied by a terminating radical; and
wherein said surfactant component is present in an amount effective to form a stable emulsion of said oil phase in said water phase.

2. A composition according to claim 1 wherein the coated pigment comprises 2 to 40 wt% of the emulsion.

3. A composition according to claim 1 or 2 wherein the aqueous phase comprises 25 to 80 wt% of the composition.

4. A composition according to claim 1 or 2 wherein the oil phase comprises 10 to 80 wt% of the composition.

5. A composition according to any of claims 1 to 4 wherein the pigment is one or more substances selected from talc, mica, titanium dioxide, ferric oxide, ferrous oxide, kaolin, ultramarine, chromium oxide, chromium hydroxide, zinc oxide, silica, manganese violet, or organic pigments or fillers.

6. A composition according to any of claims 1 to 5 which includes one or more cosmetically acceptable components dissolved in the oil phase.

7. A composition according to any of claims 1 to 6 which includes one or more cosmetically acceptable components dissolved in the aqueous phase.

8. A composition according to any of claims 1 to 7 which includes a second organic surfactant which is silicone free and wherein the surfactants are present in a combined amount effective to form a stable emulsion.

9. A composition according to any of claims 1 to 8 wherein the coated pigment has the following formula
P-[-O-Si-[-(OSiA₂)₀₋₁₀₀-A]₃]₁₋₁₀₀
wherein each of the oxygen atoms at the left end of the formula is attached to an atom P in the pigment surface; and each A is an alkyl or alkenyl group having up to 30 carbon atoms.

10. A composition according to any of claims 1 to 8 wherein the coated pigment has the following formula:
P-O-(Si(CH₃)₂O)ₚ-Si(CH₃)₃
wherein p is 1-100, and P is an atom in the pigment surface.

11. A composition according to any of claims 1 to 8 wherein the coated pigment has the following formula:
(CH₃)₃SiO-[Si(CH₃)(-OP)-O-Si(CH₃)(-O-P)-O-]₁₋₅₀-Si(CH₃)₃
wherein P is an atom in the pigment surface, and in which each of the up to 100 repeating (Si-O) units is bonded through an oxygen atom to the pigment surface.

## Patentansprüche

1. Kosmetische Zusammensetzung, die eine Öl-in-Wasser-Emulsion ist, umfassend
a) eine Ölphase, die umfaßt
i) ein beschichtetes Pigment bestehend im wesentlichen aus fein verteilten Pigmentteilchen, deren Oberflächen chemisch gebunden sind an Polysiloxan und mit demselben stofflich vollständig beschichtet sind, wobei die Beschichtung die Teilchen hydrophob macht, und
ii) eine Silikonkomponente ausgewählt von einem Dimethylpolysiloxan mit der Formel (CH₃)₃SiO(Si(CH₃)₂O)_{d}-Si(CH₃)₃, worin der Polymerisationsgrad d dergestalt wirkt, daß die Flüssigkeit eine Viskosität von 0,65 bis eine Million mm²/s (Centistokes) bei 25°C erhält; und/oder Cyclomethicon mit einem Polymerisationsgrad (D) von 3 bis 6 Grundeinheiten der Formel -(Si(CH₃)₂O)_{D}-; und/oder Organopolysiloxan mit der Formel
X(CH₃)₂SiO-Y-Si(CH₃)₂X
worin X eine Alkyl- oder Alkoxygruppe mit 1 bis 30 Kohlenstoffatomen ist und Y eine Kette von 1 bis 100 Grundeinheiten (Si-O) ist enthaltend 1 bis 100 Einheiten der Formel (-Si(R₁)(R₂)O-) und 0 bis 100 Einheiten der Formel (Si(R₃)(R₄)O), worin R₁, R₂, R₃ und R₄ jeweils eine Alkylgruppe sein kann enthaltend 2 bis 30 Kohlenstoffatome, Phenyl, oder Phenyl, das an das Si-Atom gebunden ist durch eine Vinylgruppe einer 1 bis 3 Kohlenstoffatome langen Alkylenbrücke; worin R₁ und R₃ jeweils auch -CH₃ sein kann, und R₁ und R₂ jeweils auch eine Trimethylsiloxy-Gruppe sein kann; und Mischungen davon;
b) eine wäßrige Phase;
c) eine oberflächenaktive Komponente, die ein oder mehrere Polydiorganosiloxan-Polyoxyalkylen-Copolymere umfaßt, enthaltend mindestens ein Polydiorganosiloxan-Segment bestehend aus Siloxan-Einheiten mit der Formel:
R_{b} SiO_{(4-b)/2}
worin b einen Wert von 0 bis einschließlich 3 hat, wobei durchschnittlich etwa zwei R-Radikale pro Silicium in dem Copolymer sind, und R ein Radikal darstellt, das ausgewählt ist von Methyl, Ethyl, Vinyl, Phenyl oder einem bivalenten Radikal von 2-6 Kohlenstoffatomen, die ein Polyoxyalkylen-Segment an das Polydiorganosiloxan-Segment binden, wobei mindestens 95% aller R-Radikale Methyl sind; und enthaltend mindestens ein Polyoxyalkylen-Segment mit einem durchschnittlichen Molekulargewicht von weniger als 5000 und bestehend aus 0 bis 50 Mol-% Polyoxypropylen-Einheiten und 50 bis 100 Mol-% Polyoxyethylen-Einheiten, wobei mindestens ein Endteil des besagten Polyoxyalkylen-Segments an besagtes Polydiorganosiloxan-Segment gebunden ist, wobei jeder nicht an besagtes Polydiorganosiloxan-Segment gebundene Endteil des besagten Polyoxyalkylen-Segments durch einen Abschlußrest befriedigt wird; und
worin besagte oberflächenaktive Komponente in einer wirksamen Menge vorhanden ist, daß eine stabile Emulsion der besagten Ölphase in besagter Wasserphase gebildet wird.

2. Zusammensetzung nach Anspruch 1, worin das beschichtete Pigment 2 bis 40 Gewichts-% der Emulsion umfaßt.

3. Zusammensetzung nach Anspruch 1 oder 2, worin die wäßrige Phase 25 bis 80 Gewichts-% der Zusammensetzung umfaßt.

4. Zusammensetzung nach Anspruch 1 oder 2, worin die Ölphase 10 bis 80 Gewichts-% der Zusammensetzung umfaßt.

5. Zusammensetzung nach einem der Ansprüche 1 bis 4, worin das Pigment aus einer oder mehreren Substanzen besteht ausgewählt von Talk, Mika, Titandioxid, Eisen(III)-oxid, Eisen(II)-oxid, Kaolin, Ultramarin, Chromtrioxid, Chrom(II)-hydroxid, Zinkoxid, Siliciumdioxid, Manganviolett oder organischen Pigmenten oder Füllmitteln.

6. Zusammensetzung nach einem der Ansprüche 1 bis 5, die einen oder mehrere kosmetisch annehmbare Bestandteile, die in der Ölphase gelöst sind, enthält.

7. Zusammensetzung nach einem der Ansprüche 1 bis 6, die einen oder mehrere kosmetisch annehmbare Bestandteile, die in der wäßrigen Phase gelöst sind, enthält.

8. Zusammensetzung nach einem der Ansprüche 1 bis 7, die einen zweiten organischen oberflächenaktiven Stoff enthält, der silikonfrei ist, und worin die oberflächenaktiven Stoffe in einer wirksamen Gesamtmenge vorhanden sind zur Bildung einer stabilen Emulsion.

9. Zusammensetzung nach einem der Ansprüche 1 bis 8, worin das beschichtete Pigment die folgende Formel hat:
P-[-O-Si-[-(OSiA₂)₀₋₁₀₀-A]₃]₁₋₁₀₀
worin jedes Sauerstoffatom am linken Ende der Formel an ein Atom P in der Pigmentoberfläche gebunden ist; und jedes A eine Alkyl- oder Alkenylgruppe mit bis zu 30 Kohlenstoffatomen ist.

10. Zusammensetzung nach einem der Ansprüche 1 bis 8, worin das beschichtete Pigment die folgende Formel hat:
P-O-(Si(CH₃)₂O)ₚ-Si(CH₃)₃
worin p 1-100 und P ein Atom in der Pigmentoberfläche ist.

11. Zusammensetzung nach einem der Ansprüche 1 bis 8, worin das beschichtete Pigment die folgende Formel hat:
(CH₃)₃SiO-[Si(CH₃)(-OP)-O-Si(CH₃)(-O-P)-O-]₁₋₅₀-Si(CH₃)₃
worin P ein Atom in der Pigmentoberfläche ist, und worin jede der bis zu 100 Grundeinheiten (Si-O) über ein Sauerstoffatom an die Pigmentoberfläche gebunden ist.

## Revendications

1. Composition cosmétique qui est une émulsion d'huile dans l'eau comprenant:
a) une phase huileuse qui comprend
i) un pigment revêtu consistant essentiellement de particules finement divisées de pigment dont les surfaces sont chimiquement liées à, et physiquement complètement recouvertes par du polysiloxane, lequel revêtement rend les particules hydrophobes, et
ii) un composant de silicone choisi parmi un diméthyl polysiloxane ayant la formule:
(CH₃)SiO(Si(CH₃)₂O)_{d}-Si(CH₃)₃
dans laquelle le degré de polymérisation d est efficace pour donner au fluide une viscosité de 0.65 à 1 million mm²/s (centistokes) à 25°C; et/ou une cyclométhicone ayant un degré (D) de polymérisation de 3 à 6 unités répétées de formule -(Si(CH₃)₂O)_{D}-; et/ou un organopolysiloxane ayant la formule:
X(CH₃)₂SiO-Y-Si(CH₃)₂X
dans laquelle X est un radical alkyle ou alkoxy ayant de 1 à 30 atomes de carbone, et Y est une chaîne de 1 a 100 unités répétées (Si-O) contenant 1 à 100 unités de formule (-Si(R₁)(R₂)O-) et 0 à 100 unités de formule (Si(R₃)(R₄)O) dans laquelle chacun des radicaux R₁, R₂, R₃, R₄, peut être un alcoyle contenant 2 à 30 atomes de carbone, un phényl ou un phényl lié à l'atome de Si par un groupe vinyle d'un pont alkyène de 1 à 3 atomes de carbone de long; dans laquelle chacun des radicaux R₁ et R₃ peut également être -CH₃, et chacun des radicaux R₁ et R₂ peut également être un triméthylsiloxy; et les mélanges de ceux-ci;
b) une phase aqueuse,
c) un composant tensio-actif qui comprend un ou plusieurs copolymères de polydiorganosiloxane-polyalkoxyalkylène contenant au moins un segment polydiorganosiloxane consistant d'unités siloxane ayant la formule:
R_{b} SiO_{(4-b)/2}
dans laquelle b a une valeur de 0 à 3 inclus, étant présente une moyenne d'environ deux radicaux R par silicone dans le copolymère, et R représente un radical choisi parmi méthyl, éthyl vinyle, phényl ou un radical divalent de 2 à 6 carbones, liant un segment polyoxyalkylène au segment polydiorganosiloxane, au moins 95% de tous les radicaux R étant méthyl; et contenant au moins un segment polyoxyalkylène ayant en moyenne un poids moléculaire de moins de 5 000 et consistant de 0 à 50 mol/pourcent d'unités de polyoxypropylène et de 50 à 100 mol/pourcent d'unités de polyoxyéthylène, au moins une portion terminale dudit segment polyoxyalkylène étant lié audit segment polydiorganosiloxane, toute portion terminale dudit segment polyoxyalkylène n'étant pas lié au segment polydiorganosiloxane comportant un radical de terminaison; et dans laquelle ledit agent tensio-actif est présent en quantité efficace pour former une émulsion stable de ladite phase huileuse dans ladite phase aqueuse.

2. Composition selon la revendication 1, dans laquelle le pigment revêtu comprend 2 à 4% en poids d'émulsion.

3. Composition selon la revendication 1 ou 2, dans laquelle la phase aqueuse comprend 25 à 80% en poids de composition.

4. Composition selon la revendication 1 ou 2, dans laquelle la phase huileuse comprend 10 à 80% en poids de composition.

5. Composition selon l'une quelconque des revendications 1 à 4, dans laquelle le pigment est une ou plusieurs substances choisies parmi le talc, le mica, le dioxyde de titane, l'oxyde ferrique, l'onde ferreux, le kaolin, l'ultramarine, l'oxyde de chrome, l'hydroxyde de chrome, l'oxyde de zinc, la silice, le violet de manganèse, ou des pigments organiques ou des charges.

6. Composition selon l'une quelconque des revendications 1 à 5, qui comprend un ou plusieurs composant(s) cosmétiquement acceptable(s) dissout(s) dans la phase huileuse.

7. Composition selon l'une quelconque des revendications 1 à 6, qui comprend un ou plusieurs composant(s) cosmétiquement acceptable(s) dissout(s) dans la phase aqueuse.

8. Composition selon l'une quelconque des revendications 1 à 7, qui comprend un deuxième tensio-actif organique ne contenant pas de silicone, mais dans laquelle les tensio-actifs sont présents eu quantité combinée efficace pour former une émulsion stable.

9. Composition selon l'une quelconque des revendications 1 à 8, dans laquelle le pigment recouvert a la formule suivante:
P-[-O-Si-[-(OSiA₂)₀₋₁₀₀-A]₃]₁₋₁₀₀
dans laquelle chacun des atomes d'oxygène à l'extrémité gauche de la formule est lié à un atome P dans la surface du pigment; et chaque A est un radical alcoyle ou un groupe alcényle ayant jusqu'à 30 atomes de carbone.

10. Composition selon l'une quelconque des revendications 1 à 8, dans laquelle le pigment revêtu a la formule suivante:
P-O-(Si(CH₃)₂O)ₚ-Si(CH₃)₃
dans laquelle p varie de 1 à 100, et P est un atome dans la surface du pigment.

11. Composition selon l'une quelconque des revendications 1 à 8, dans laquelle le pigment revêtu a la formule suivante:
(CH₃)₃SiO-[Si(CH₃)(-OP)-O-Si(CH₃)(-O-P)-O-]₁₋₅₀-Si(CH₃)₃
dans laquelle P est un atome dans la surface du pigment, et dans laquelle chacune des unités de répétition (Si-O) jusqu'à 100, est liée par un atome d'oxygène à la surface du pigment.
